# EUROPEAN PATENT APPLICATION

(11) **EP 3 698 720 A1**
(43) Date of publication of application: **26.08.2020**
(21) Application number: 19158009.1
(22) Date of filing: 19.02.2019
(51) Int. Cl.: A61B 6/04, A61B 5/055

(54) **HEAD- AND ARM-REST DEVICE FOR USE IN MEDICAL SCANNING**

(71) Applicant: Herlev Hospital, 2730 Herlev (DK)
(72) Inventor: Tietgen, Nina, 2730 Herlev (DK); Nydahl, Aske, 2730 Herlev (DK); Hermansen, Jonas, 2730 Herlev (DK)
(74) Representative: Høiberg P/S

(57) **Abstract**

The invention relates to a head- and armrest device attachable to a table for supporting head and arms of a subject lying on the table during medical imaging, comprising an adjustable headrest configured to support the head of the subject in a first plurality of angular and/or vertical positions relative to a plane defined by the table and an adjustable armrest adjacent to the headrest and configured to support one or both arms of the subject in a second plurality of angular and/or vertical positions relative to said plane, wherein the headrest and the armrest are adjustable independent of each other.

## Description

### Field of invention

The invention relates to an adjustable device attachable to a table for supporting a patient lying on the table during medical imaging.

### Background of the Invention

When performing medical scans such as CT, PET/CT or MRI scans the patient has to lie in a fixed position for a given period of time, depending on the type of the scan and the area of scanning. Especially in scan types which take a long time such as MRI, PET or combined PET/CT scans, it can be very difficult for the patient to keep their head and arms in a fixed position in the duration of the scan. In addition the position in which the patient is asked to remain is often awkward and it is exhausting to remain in this fixed position for long periods of time. For example, a patient having a PET/CT scan of the neck-area will often have to place the arms above the head. It is hence crucial to provide proper support for the patient's arms and head, during the long medical scan, to ensure that the patient lies still during the entire period of the scan or scans. To ensure this the patient is often supported by a headrest and/or armrest which support the patient's head and/or arms in a desired position. The arms can also be supported by pillows or similar, however stacking pillows to gain the best possible comfort for the patient is often tedious, inefficient and the result not very good. Furthermore it is not a reproducible positioning of the patient.

It is of crucial importance that a person lies still during the scan or scans, especially for dual modality scans in which two different images types will be co-registered and shown on top of each other, for example where one serves as an anatomical image (CT/MRI) and the other as a functional image (PET/MRI). This is the case for instance with PET/CT scans. In PET/CT scans the relatively fast CT scan provides a map of the body whereas the PET scan displays the distribution of the injected radio-tracer, i.e. ¹⁸F-FDG, which as a glucose analogue indicates cellular glucose metabolism, which can be an indicator in some types of cancer. The co-registration of the PET and CT images serves two purposes, firstly CT allows for a precise location of the PET signal to obtained, secondly the CT image serves as input in the attenuation correction of PET images, leading to a better image quality. Hence if the patient has moved during or between the two scans, the physician might mistakenly believe the tumour to be in a wrong part of the body if the overlay between the two scans is not perfect due to patient movement, similarly the attenuation correction of the PET image with a misaligned CT can cause image artefacts which may lead to misdiagnosis.

US 4,616,814 discloses a headrest which is adjustable about an axis perpendicular to the long axis of the patient table and vertically displaced from the table plane.

US 2013/028391 discloses a headrest which is supported directly on the patient table, or possibly on an extension plate, where the head tilt can be adjusted by introducing wedges underneath the head support. This head support may be combined with a static armrest which can be attached directly to the top of the headrest.

US 2014/163354 discloses a combined head- and armrest taking up space on the table or on an extension table piece. The arms are not supported underneath but only by holding or fixating the hands in a given position.

US 2008/178893 discloses a headrest system in which the head position can be vertically adjusted from the level of the table.

### Summary of the invention

One purpose of the present disclosure to provide a head-rest and arm-rest that in combination provide for improved patient support during medical imaging. The present disclosure therefore relates to a head- and armrest device attachable to a table for supporting head and arms of a subject lying on the table during medical imaging procedures. One embodiment of the head- and armrest device comprises an adjustable headrest configured to support the head of the subject, preferably in a first plurality of angular and/or vertical positions relative to a plane defined by the table, and an adjustable armrest adjacent to the headrest and configured to support one or both arms of the subject, preferably in a second plurality of angular and/or vertical positions relative to said plane. For improved patient comfort and support and for improved medical scanning image quality the headrest and the armrest of the presently disclosed head- and arm-rest device are adjustable independently of each other. Independent adjustment provides for an adaptation to the individual patient for improved comfort, but also such that medical personnel can adjust the presently disclosed device for optimal anatomical positioning of the patient during medical imaging / scanning. This minimizes the unintentional movement the patient may do during a medical scan which may result in movement of the sections of the body relevant to the scan. Even small movements of the arm(s) will result in relatively drastic movements of the thorax, possibly leading to misalignment of the images.

The head- and armrests of the present invention may be adjusted through a tilt-like motion in which the headrest and/or armrest is rigidly rotated about one or more axes. Also a vertical, elevator-like movement of the headrest and/or armrest may be possible, i.e. a movement which is vertical / elevator-like when the head- and arm-rest device is attached to the table.

The headrest and armrest of the presently disclosed device may be individually adjusted to ensure the correct positioning of the head and arm(s) of the subject for any given medical scan which requires fixation or support of the head and/or arm(s) for the duration of the scan. Hence the device is especially useful in the case of dual modality scans. The present disclosure enables the ideal positioning of head and arms by a single device which ensures a fast and comfortable fixation or support of head and arms in a position which can be customized to the individual patient for the given medical imaging procedure.

Thus, for the presently disclosed device it is possible to provide support for the arm or arms and head of a patient in any desirable combination of head and arm(s) positions. It allows the arms to be fixed at any desirable position which can be adjusted to any given purpose. This enables positioning of the head and arms in a position that optimizes the imaging of the regions in the body which is of interest in a particular scan. Furthermore it allows fixation of the arms and head and, through this, also the body of the patient which minimizes the movement during a scan and in the case of a dual modality scan, also between two scans.

The device is also advantageous in cases where one needs to acquire a series of scans where the patient will need to have the arm/arms and/or head in one position in one scan and in a different position in another scan. For example a patient may have to keep the arms above the head in one scan and along the sides of the body in another scan. Without the device of the present disclosure these different needs may result in the staff of the scan facility having to change from one type of head and/or armrest to another between scans. The patient would then need to wait and possibly leave the table, while the other head and/or armrest is mounted on the table. This will lead to some waiting time for the patient in which the scanner is not running, and the duration of the medical imaging procedure will be longer than the actual effective scan time because the medical personal will need time to change the equipment attached to or supported by the table. Ultimately this means that fewer patients will be able to get scanned per day, simply because a procedure takes an unnecessary long time. By using the device of the present disclosure the same head- and/or armrest system can be used to support the head when the arms are placed along the body as well as support the head and arms when the arms are placed above the head. In addition, simply being able to change the position of the attached headrest and armrest will enable the same support device to be used at many different imaging procedures and will eliminate or minimize the need for change of support device (head and/or armrest) between two scans or between two patients.

### Description of the drawings

The invention will in the following be described in greater detail with reference to the accompanying drawings:
Fig 1 shows a top-view sketch of one embodiment of the presently disclosed head- and armrest.
Fig 2 shows a side-view sketch of one embodiment of the presently disclosed head- and armrest.
Fig 3 shows a bottom-view sketch of one embodiment of the presently disclosed head- and armrest.
Fig. 4 shows an image of one embodiment of the presently discloses head- and armrest.
Fig. 5 shows an image of the same embodiment of the presently disclosed head- and armrest as in Fig. 4.
Fig. 6: shows one embodiment of a person lying in the presently disclosed head- and armrest.
Fig. 7 shows the embodiment of Fig 6 with a person supported by the head- and armrest at different angles.
Fig. 8 shows the embodiment of Fig 6+7 with a person supported by the head- and armrest at different angles.
Fig. 9 shows the embodiment of Fig 6-8 showing the attachment mechanism in a top view (top image) and an image from below the table (lower image).
Fig. 10 shows the embodiment of Fig 6-9 with a full setup ready for a patient scan.

### Detailed description of the invention

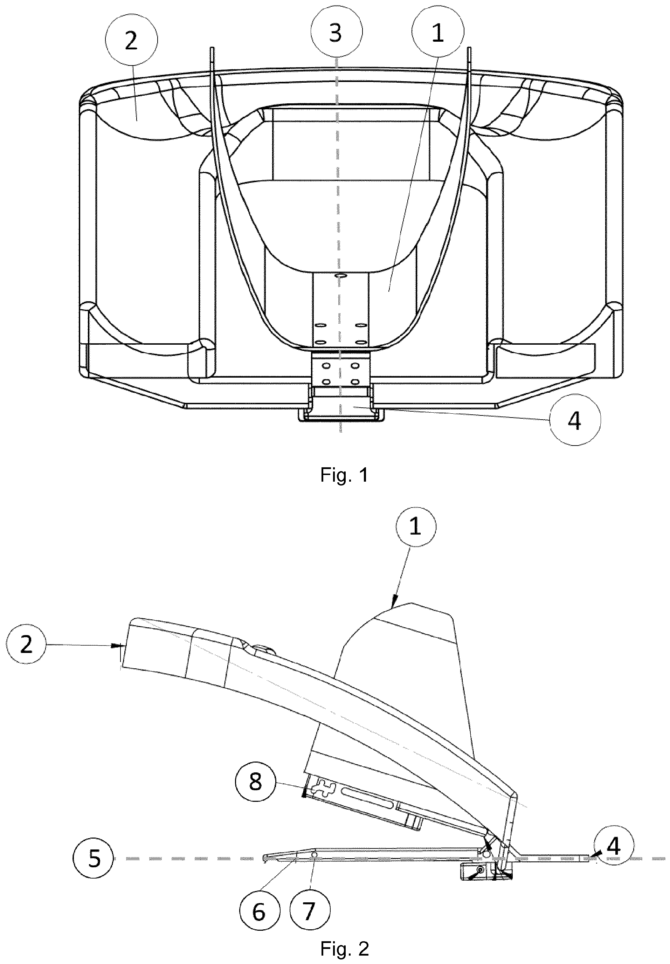

The presently disclosed head- and armrest device is preferably symmetric about a mirror plane passing through the middle of the headrest and the middle of the armrest. One embodiment of such a symmetry about a mirror plane is shown in Fig. 1 about the plane indicated by 3 in the figure. In the preferred embodiment the device comprises an attachment mechanism to attach the head- and armrest to the table where said attachment mechanism is placed in the end of the device configured to face the table. The attachment mechanism may be in the form of a rod structure configured to attach the head- and armrest device to the table. One embodiment of such an attachment mechanism is shown at 4 in Figures 1-3. The attachment mechanism may be passing through the mirror plane. Furthermore the attachment mechanism may itself be symmetric so that also the attachment mechanism is mirrored by the mirror plane. The attachment mechanism may allow the device to be situated at an adjustable distance to the table or a fixed distance which can also mean a zero-distance in the case of direct, physical contact between the headrest and/or armrest of the device and the edge of the table.

The attachment may be made to the table in a manner so that the device is attachable to the table as an extension piece. Hence, the head- and armrest will not take up space on the table itself. The patient may for instance lie with the shoulders supported by the table and the head and arms outside of the table area resting in the head- and armrest.

By being attached as an extension piece the device is not lying on the table, nor is it supported underneath by an extension table attached to the table as an extension to the table length. This is an important feature as some scanners do not allow scans of the entire length of the table, which hence limits the scan-able area of a person lying on the table. Adding an extension table or moving the patient further down the table will limit the areas of the body available for scan. Some existing head- and/or armrests are placed directly on the table. This means that the device is supported by an underlying support and takes up space on the table by lying directly on the table or on the extension of the table. Such head and/or armrests limits the available scan area for the scanners not able to scan the full table length. By positioning the head outside the table area, as in this embodiment of the head- and armrest device, a larger part of the body will be able to enter the scanner. Hence mounting the device as an extension to the table allows for maximization of the available scan-regions of the body of the patient when using a scanner type of limited scan-length.

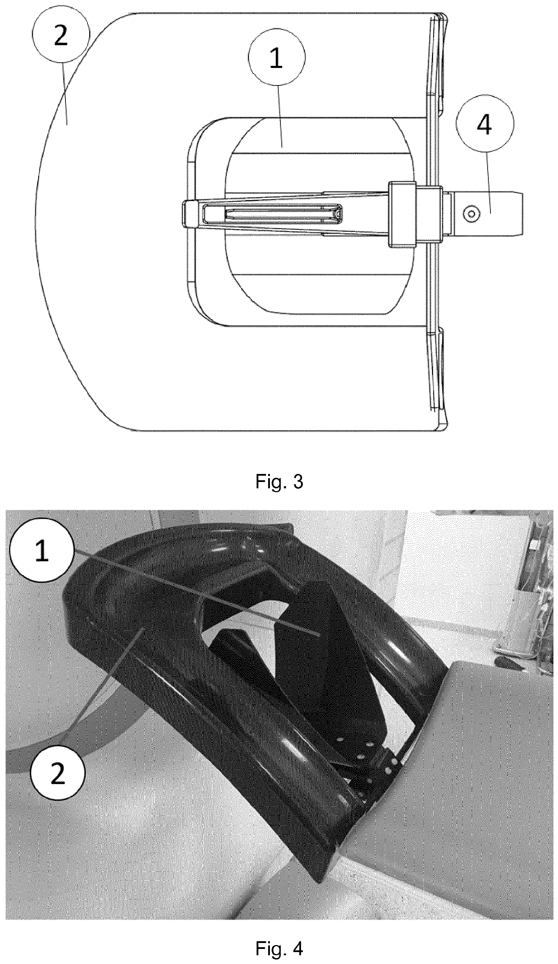

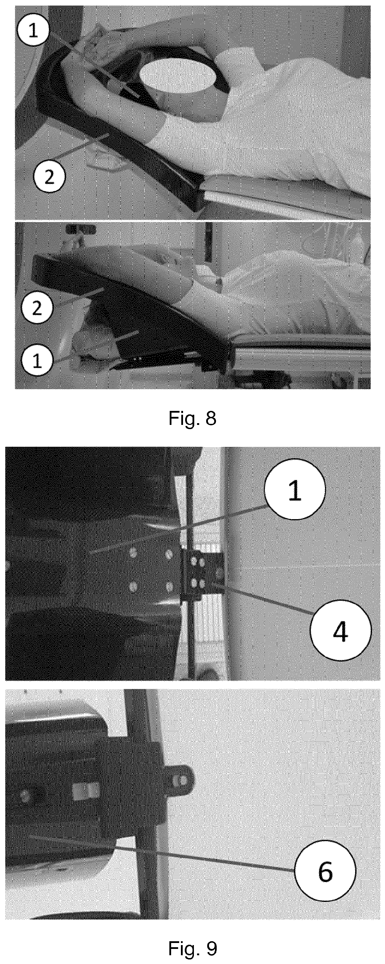

In one embodiment the attachment mechanism of the present disclosure may happen through an attachment device such as a rod, a board, a tongue or a stick. The attachment device may be formed as a rectangular shaped board such as the one shown at 4 in Figure 3. The attachment mechanism may hence happen through an accessory port underneath the table used to attach add-ons to the table. The attachment device may then be placed inside a socket-like structure of the accessory port and fasten by a fixation screw which may be a spring-loaded screw fastening the position of the attachment device inside the accessory port. One embodiment of such an attachment is shown in Figure 9.

The headrest and the armrest, of the presently disclosed device, may be individually adjustable about the same axis or the headrest and armrest may be individually adjustable about two different axes. In the preferred embodiment the headrest and/or the armrest are individually adjustable about an axis perpendicular to the mirror plane of the device or individually adjustable about two different axes both perpendicular to the mirror plane of the device. For example could the headrest be adjusted about one axis, perpendicular to the mirror plane of the device 3 but not contained in the plane of the attachment mechanism 5 the armrest may then be adjustable about another axis also not contained in the plane of the armrest mechanism but perpendicular to the attachment plane. Hence the axis of adjustment of the headrest and armrest may be vertically displaced relative to each other or may be two different axes within the same plane or they could be adjustable about the same axis.

In one embodiment the adjustment of the armrest may happen by a hinge-like structure, which allows for a smooth adjustment of the armrest. Once the desirable position of the armrest is found the armrest can be fastened in a given position by tightening a screw pressing the two parts of the hinge-like structure together, preventing them from moving relatively to each other and by then fixing the armrest position. In one embodiment the head- and/or armrest is adjustable using a stepped fastening mechanism. In such a system the headrest and/or armrest may be positioned in a discrete set of positions, e.g. by a stepped adjustment. This may be provided by a mechanism such as the one illustrated in Figure 2. The positioning stick 6 may then be adjustable and moved towards the headrest 1 and the pin 7 of the positioning stick 6 may be passing through the jagged hole 8 where the pin can be placed in any of the indentations to fixate the headrest in this particular position. Hence the example shown in Fig. 2 has four different positions of the headrest.

In another embodiment the head- and/or armrest is adjustable using a step-less fixation mechanism. In such a system the head- and/or armrest may be positioned at any position in a continuous range of positions.

The individual adjustments of the headrest and the armrest are important to ensure support and fixation of the patient's head and arms in the optimal position for a particular medical scan. In one embodiment the adjustment of the headrest and/or armrest can adjusted to both neutral, negative or positive angles and/or vertical positions relative to horizontal levels defined by their respective rotation / fixation axes, which may be coinciding or horizontally and/or vertically displaced as previously indicated.

Hence, in one embodiment the headrest may be rotationally adjustable to an inclination (i.e. angle) below zero degrees relative to horizontal, i.e. such that the head of the patient is bent slightly backwards when supported by the headrest. Preferably the headrest may be rotationally adjustable to an angle at least below -5 degrees, more preferably at least below -10 degrees relative to horizontal, possibly even below -15 degrees. Likewise the headrest may be rotationally adjustable to a positive inclination, i.e. to an angle of at least +10 degrees relative to horizontal, more preferably at least 20 degrees, most preferably at least 30 degrees relative to horizontal, i.e. such that the head of the patient is bent slightly forward.

The same may be possible for the armrest, which can also be adjusted between 0, -5, - 10 or -15 degrees and 10, 20 or 30 degrees relative to horizontal. In fig. 2 the horizontal plane of the armrest is exemplified by the dashed line 5.

I.e. a patient lying with the head and possibly arms in the disclosed device (lying on the back on the table) will have the head tilting backwards when the headrest is in a negative angle/vertical position. The head is in a neutral position when it is in level with the attachment mechanism and when the orientation is horizontal. In a positive position of the head relative to the attachment mechanism the neck of the patient is bending upwards when lying on the back. The same is possible for the arms, which may be laid back into a level below the attachment mechanism, hence the arms will be further back than the back of the patient, lying on the back on the table. The arms can also be supported in a neutral or upright position, wherein the latter refers to the arms being supported at a level above the back of the patient lying on the table.

In one embodiment the head-rest is shaped in a U-shape, in which the curvature of the U-shaped headrest is configured to support the back of the head of said subject. Hence the shape will roughly follow the shape of the back of the head which will improve the comfort. The patient, lying on the back will have the head supported from below when placed in the U-shaped headrest.

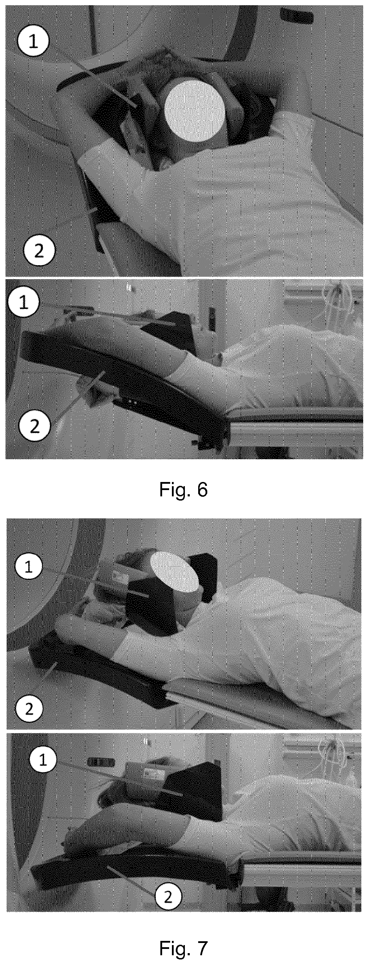

In the preferred embodiment the straight sides of the headrest are configured to extend further than the head of the patient. For example, when a patient lies with his or her head in the headrest at a neutral position (horizontal), lying on the back on the table to which the device is attached, the chin bones of the patient should not be further up than the sides of the headrest. One example of this is shown in the side views of Figure 6-8. This provides a gentle fixation of the head which helps the patient lie still during the scan. Unlike other headrests not having these high sides the patient is guided not to turn the head. Furthermore if the device is constructed in a non-transparent material and the sides are constructed without holes the patient is not tempted to slightly turn the head to get a glimpse of what is happening to either side. In another embodiment the straight sides of the headrest are long enough to enable the fixation of wedges between the headrest and the head of a subject. This is a way to introduce further immobilization of the patient's head. This can be done, for instance, by soft, foam wedges which may be wedged in between the sides of the headrest and the sides of the persons head such as at the cheeks, cheekbone or at the temples.

In one embodiment the armrest and the headrest are both fastened to the table. In another embodiment the armrest is fastened to the table and the headrest is fastened to the armrest. In yet another embodiment the headrest is fastened to the table and the armrest is fastened to the headrest. From this the attachment mechanism can either be that the headrest and armrest are individually attached to the table but it is also possible that only one of the components, the headrest or the armrest, is attached directly to the table, and then the other component is attached to the component which is attached to the table.

It is important to manufacture the device in a material which has little or no influence on the contrast of the scan image, and will introduce less noise. This helps generating the best possible image in which diagnostics can be reliably carried out. Another demand on the choice of material is that the material has to be quite hard and rigid to be able to steadily support the head and arms of a patient. For this purpose carbon fiber makes for an excellent choice. Hence, in the preferred embodiment of the present disclosure the head- and armrest device is fully of partly fabricated of carbon fiber or plastic.

In one embodiment the cross section of the armrest, cut through the mirror plane of the device, is U shaped. One embodiment of a U-shaped armrest is shown in Figure 1. By a U-shaped armrest, the arm or arms of the patient, when lying in the device with the arms in the armrest, will be supported in a more comfortable way in which the armrest follows the curvature or the arm. The U-shape ensures the stability of the armrest as it strengthens the overall structure. In addition the arms of the patient will be somewhat sunken into the U-shape of the armrest so that the sides of the armrest curvature help the arm stay in the right place. The armrest may hence be shaped as a hollow shell in which the arm fits in the cavity of the shell. In this manner the armrest may support the entire arm or almost the entire arm by a direct support underneath the arm. The armrest may be shaped to surround the headrest which still allows adjustment of the headrest within the line of adjustment of the headrest. In this manner the patient arms will be wrapped around the head so that the underarms of a patient, using the device, are crossing and lying on top of each other or next to each other at the top of the head.

Some embodiments of the armrest surrounding the headrest are shown in Fig. 1 and Figs. 4-5. In another embodiment armrest have a flat structure instead of a U-shaped structure which means that the cross section of the armrest, cut through the mirror plane of the device, is flat instead of U-shaped.

In one embodiment the head- and armrest device is configured such that the arms can be fastened in the armrest by a strap. This can be an actual strap, a string, a cord or basically any material that can be used to wrap round the arm and armrest to secure the position of the arm in the armrest. In another embodiment the head- and armrest device comprises a strap configured to fasten the arms in the armrest. In this embodiment the strap is an integrated part of the device. The strap may serve the same purpose as in the previous embodiment, namely that the arm or arms can be further secured in the armrest, so that the arm or arms can be immobilized and fixated during the scan.

In one embodiment the head- and armrest is configured such that the head can be fastened in the headrest by a strap. The head may be fastened using a string or a cord or basically any material that can be used to wrap round the head and headrest to secure the position of the head in the headrest. In another embodiment the head- and armrest device comprises a strap configured to fastening the head in the headrest. The strap again serves the same purpose as in the previous statement embodiment, namely that the head can be further secured in the headrest, so that the head can be immobilized and fixated during the scan.

In the preferred embodiment the armrest and/or headrest is lined with a soft material such that the comfort of the device is improved. The soft material may be a pillow (such as the one supporting the backside of the head of the patient in Fig. 6-8) or a piece of foam and could be fasten to the device by the use of Velcro, scotch tape, duct tape or strings through strategically placed holes made in the head- and armrest device. It would be preferable to make the fixation in a way such that the linen can be easily removed and replaced so that each patient can get a clean set of linen in the device. The linen may be washable or disposable.

In one embodiment of the present disclosure the armrest and headrest can be individually detached from each other. This may be preferable in the process of cleaning the device. The head and armrest device may also, in another embodiment, be an integrated part in which the headrest and armrest cannot be taken apart.

In another embodiment the armrest and headrest of the presently disclosed device can be attached to the table independently of each other. This may be practical for example if the headrest and armrest can be individually detached from each other and each part can be individually attached to the table, then it is possible to use just the armrest or just the headrest and simply remove the other part completely. This may optimize the usage of the device in a brain scan for instance, where the removal of the armrest will minimize the noise of the images, and provide a better image. If, in another embodiment, the headrest and armrest cannot be separated and it is necessary to only use the headrest, the armrest may be left empty and the scan be carried out in this manner. The armrest may then be set in the angle that disturbs the scan images the least.

### Examples

Fig 1 shows a sketch of one embodiment of the present disclosure in a top view where the headrest 1 is U-shaped and the armrest 2 is surrounding the headrest. The attachment mechanism 4 is placed in the end of the head- and armrest configured to face the table which means the section configured to accommodate the lower part of the head and the upper arms of the patient. The device is symmetric about a mirror plane indicated by the dashed line 3.

Fig 2 shows a sketch of one embodiment of the present disclosure in a side view indicating the headrest 1 armrest 2 the plane of attachment indicated by the dashed line 5 and the attachment mechanism which may be a rod 4. The positioning stick 6 has a small pin 7 which may be inserted in the jagged hole 8 in the part of the headrest 1 below the U-shaped shell of the headrest. The placement of the pin in the jagged hole may then lock the headrest in a given position. It should be understood that the positioning stick 6 is attached through the small pin 7 into the jagged hole 8 to the headrest 1 and hence positioning stick 6 and the headrest 1 are never apart in the working device, even though shown in this sketch. In the embodiment of this figure the headrest will hence have four possible positions as the jagged hole has four indentations.

Fig 3 shows a sketch of one embodiment of the present disclosure in a view from below. The device comprises the headrest 1 armrest 2 and attachment device 4.

Fig. 4 shows one embodiment of the present disclosure in which the headrest 1 is U-shaped and the armrest 2 is surrounding the headrest and comprises a cavity for placing the arms of a patient.

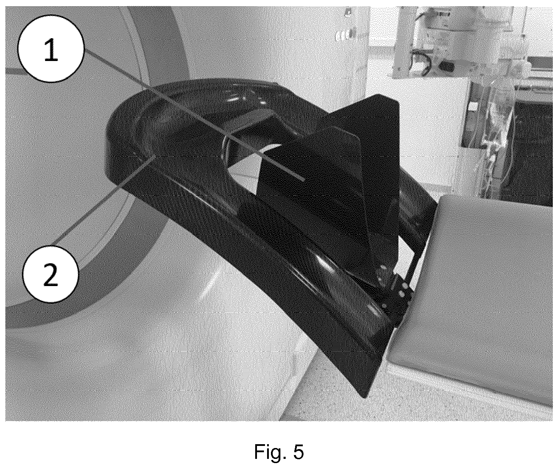

Fig. 5 shows the same embodiment of the present disclosure as Fig. 4 in which the headrest 1 has been moved (tilted) individually of the armrest 2 (relative to figure 4) to a more upright position of the headrest.
One of the great advantages of having an adjustable head and armrest is that the possibility of fixating a patient in a position which maximized the scanning sensitivity to certain regions of the body. Hence different relative positions of headrest and armrest will allow the patient to be in the optimal position for imaging a specific part of the body.

Fig. 6: shows one embodiment of the present disclosure showing how a person may lie with arms and head in the head- and armrest. In this embodiment wedges has been used to further secure the fixation of the head. The headrest 1 is furthermore lined with a pillow to increase the comfort of the device. Top figure shows a frontal image whereas the lower image shows the embodiment in a side view.

Fig. 7 shows the embodiment of Fig 6 but with the armrest 2 moved downwards to a neutral or slightly negative angle of the armrest relative to the plane of attachment and the plane of the table. The headrest is further moved up in a positive angle relative to the attachment mechanism or the table plane. Such a setup in which the head is at a high position and the arms are at a low position is for instance desirable when scanning patients with scoliosis or other complications which makes the patient unable to lying flat on the back. Top image: an image of the embodiment in a slanting angle, lower image: side view of the embodiment.

Fig. 8 shows the embodiment of Fig. 6 and Fig. 7 in which the armrest 2 is in a positive angle relative to the attachment plane or the table plane and the headrest 1 is in a slightly negative angle relative to the attachment plane or the plane of the table. Such a situation in which the head is kept at a low position and the arms at a high position is for example suitable for patients who have problems with their shoulders such asarthritis in the shoulders or has complications in their shoulders from former operation. Also Mammae-patients or patients which has recently had removal of axillary lymphatics can benefit from this positioning of arms and head. The position is also desirable for scans of thyroid area (Thyreoidea/Parathyreoidea). Top image: an image of the embodiment in a slanting angle, lower image: side view of the embodiment.

Fig. 9 shows the embodiment of Fig 6-8 showing the attachment mechanism in a top view (top image) from above the table in which the left part of the image shows the headrest 1 and the right part shows the table onto which the headrest, possibly with the armrest, is attached to the table using the attachment mechanism 4. Below is a view from below the table in which the positioning stick 6 is visible. Here the attachment board is inserted in the accessory port in the edge of the table. The table is again shown in the right part of the image

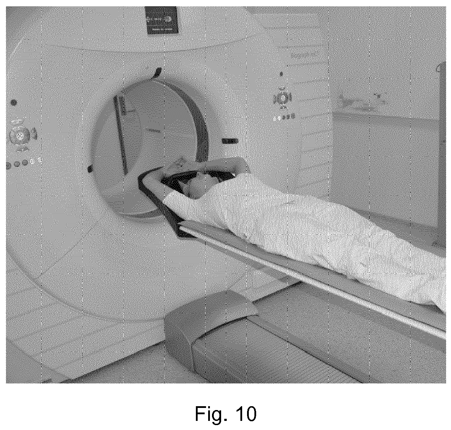

Fig. 10 shows one embodiment in which the disclosed device is attached to a table and setup to initiate a scanning in the scanning apparatus in the background.

Further details of the present invention are described by the following items:

### Items

1. A head- and armrest device attachable to a table for supporting head and arms of a subject lying on the table during medical imaging, the head- and armrest device comprising:
   - an adjustable headrest configured to support the head of the subject in a first plurality of angular and/or vertical positions relative to a plane defined by the table,
   - an adjustable armrest adjacent to the headrest and configured to support one or both arms of the subject in a second plurality of angular and/or vertical positions relative to said plane,
      wherein the headrest and the armrest are adjustable independent of each other.
2. The head- and armrest device according to item 1, wherein the device is symmetric about a mirror plane passing through the middle of the headrest and the middle of the armrest.
3. The head- and armrest device according to any of the proceeding items, comprising an attachment mechanism to attach the head- and armrest to the table where said attachment mechanism is placed in the end of the device configured to face the table.
4. The head- and armrest device according to any of the proceeding items, comprising an attachment mechanism configured to attach the head- and armrest device to the table
5. The head- and arm-rest device according to any of the proceeding items, wherein the headrest and the armrest are individually adjustable about the same axis.
6. The head- and armrest device according to any of the proceeding items,
   wherein the headrest and armrest are individually adjustable about two different axes.
7. The head- and armrest device according to any of the proceeding items,
   wherein the headrest and/or the armrest is individually adjustable about an axis perpendicular to the mirror plane of the device.
8. The head- and arm-rest device according to any of the proceeding items,
   wherein the head- and/or armrest is adjustable using a stepped fastening mechanism.
9. The head- and arm-rest device according to any of the proceeding items, wherein the head- and/or armrest is adjustable using a step-less fixation mechanism.
10. The head- and arm-rest device according to any of the proceeding items,
   wherein the adjustment of the headrest and/or armrest can happen at both neutral, negative or positive angles and/or vertical positions relative to the level of the attachment mechanism.
11. The head- and arm-rest device according to any of the proceeding items,
   wherein the headrest and/or armrest is rotationally adjustable to an inclination below zero degrees relative to horizontal, preferably the headrest is rotationally adjustable to an angle at least below -5 degrees, more preferably at least below -10 degrees relative to horizontal, possibly even below -15 degrees relative to horizontal.
12. The head- and arm-rest device according to any of the proceeding items,
   wherein the headrest and/or armrest is rotationally adjustable to a positive inclination, preferably, to an angle of at least +10 degrees relative to horizontal, more preferably at least 20 degrees, most preferably at least 30 degrees relative to horizontal.
13. The head- and arm-rest device according to any of the proceeding items,
   wherein the headrest and/or armrest is rotationally adjustable to allow an inclination of zero degrees relative to horizontal.
14. The head- and arm-rest device according to any of the proceeding items,
   wherein the device is attachable to the table as an extension piece.
15. The head- and arm-rest device according to any of the proceeding items,
   wherein the head-rest is shaped in a U-shape, in which the curvature of the U-shaped headrest is configured to support the back of the head of said subject
16. The head- and arm-rest device according to any of the proceeding items,
   wherein the straight sides of the headrest are configures to extend further than the head of the patient.
17. The head- and arm-rest device according to any of the proceeding items,
   wherein the straight sides of the headrest are long enough to enable the fixation of wedges between the headrest and the head of a subject.
18. The head- and arm-rest device according to any of the proceeding items,
   wherein the armrest and the headrest are both fastened to the table
19. The head- and arm-rest device according to any of the proceeding items, where the armrest is fasten to the table and the headrest is fasten to the armrest.
20. The head- and arm-rest device according to any of the proceeding items, where the headrest is fasten to the table and the armrest is fasten to the headrest.
21. The head- and arm-rest device according to any of the proceeding items,
   wherein the head- and arm-rest device is fully of partly fabricated of carbon fiber or plastic.
22. The head- and arm-rest device according to any of the proceeding items,
   wherein the arm-rest is shaped to surround the headrest and to support the arms in a position over the head.
23. The head- and arm-rest device according to any of the proceeding items,
   wherein the cross section of the armrest, cut through the mirror plane of the device, is U shaped.
24. The head- and arm-rest device according to any of the proceeding items,
   wherein the cross section of the armrest, cut through the mirror plane of the device, is flat.
25. The head- and arm-rest device according to any of the proceeding items, configured such that the arms can be fasten in the armrest by a strap.
26. The head- and arm-rest device according to any of the proceeding items, comprising a strap configured to fasten the arms in the armrest.
27. The head- and arm-rest device according to any of the proceeding items, configured such that the head can be fasten in the headrest by a strap.
28. The head- and arm-rest device according to any of the proceeding items, comprising a strap configured to fastening the head in the armrest.
29. The head- and arm-rest device according to any of the proceeding items,
   wherein the armrest and/or headrest is lined with a soft material such that the comfort of the device is improved.
30. The head- and arm-rest device according to any proceeding items, wherein the armrest and headrest can be individually detached from each other.
31. The head- and arm-rest device according to any proceeding items, wherein the armrest and headrest can be attached to the table independently of each other.

## Claims

1. A head- and armrest device attachable to a table for supporting head and arms of a subject lying on the table during medical imaging of said head and/or arms, the head- and armrest device comprising:
- an adjustable headrest configured to support the head of the subject in a first plurality of angular and/or vertical positions relative to a plane defined by the table, and
- an adjustable armrest adjacent to the headrest and configured to support one or both arms of the subject in a second plurality of angular and/or vertical positions relative to said plane,
wherein the headrest and the armrest are adjustable independent of each other.

2. The head- and armrest device according to claim 1, wherein the device is symmetric about a mirror plane passing through the middle of the headrest and the middle of the armrest and where the headrest and/or the armrest is individually adjustable about an axis perpendicular to said mirror plane of the device.

3. The head- and armrest device according to any of the proceeding claims, comprising an attachment mechanism configured to attach the head- and armrest device in longitudinal extension of the table.

4. The head- and arm-rest device according to any of the proceeding claims,
wherein the headrest and the armrest are individually adjustable about the same axis.

5. The head- and armrest device according to any of the proceeding claims,
wherein the headrest and armrest are individually adjustable about two different axes.

6. The head- and arm-rest device according to any of the proceeding claims,
wherein the head- and/or armrest is adjustable using a stepped fastening mechanism.

7. The head- and arm-rest device according to any of the proceeding claims,
wherein the head- and/or armrest is adjustable using a step-less fixation mechanism.

8. The head- and arm-rest device according to any of the proceeding claims,
wherein the adjustment of the headrest and/or armrest can happen at both neutral, negative or positive angles and/or vertical positions relative to the level of the attachment mechanism.

9. The head- and arm-rest device according to any of the proceeding claims,
wherein the headrest and/or armrest is rotationally adjustable to an inclination below zero degrees relative to horizontal, preferably the headrest is rotationally adjustable to an angle at least below -5 degrees, more preferably at least below -10 degrees relative to horizontal, possibly even below -15 degrees relative to horizontal.

10. The head- and arm-rest device according to any of the proceeding claims,
wherein the headrest and/or armrest is rotationally adjustable to a positive inclination, preferably, to an angle of at least +10 degrees relative to horizontal, more preferably at least 20 degrees, most preferably at least 30 degrees relative to horizontal.

11. The head- and arm-rest device according to any of the proceeding claims,
wherein the headrest and/or armrest is rotationally adjustable to allow an inclination of zero degrees relative to horizontal.

12. The head- and arm-rest device according to any of the proceeding claims,
wherein the head-rest is shaped in a U-shape, in which the curvature of the U-shaped headrest is configured to support the back of the head of said subject.

13. The head- and arm-rest device according to any of the proceeding claims,
wherein the medical imaging is selected from the group of: CT, MRI, fMRI, PET, and combinations thereof.

14. The head- and arm-rest device according to any of the proceeding claims,
wherein the arm-rest is shaped to surround the headrest and to support the arms in a position over the head.

15. The head- and arm-rest device according to any of the proceeding claims,
wherein the cross section of the armrest, cut through the mirror plane of the device, is U shaped.
